# EUROPEAN PATENT APPLICATION

(11) **EP 4 133 992 A1**
(43) Date of publication of application: **15.02.2023**
(21) Application number: 22189899.2
(22) Date of filing: 11.08.2022
(51) Int. Cl.: A61B 3/06, A61B 3/00, A61B 3/032, A61B 3/028, A61B 3/09, A61B 3/103, A61B 3/11, A61B 3/113

(54) **DETERMINING COLOR VISION ABILITY USING A VISION SCREENING DEVICE**

(30) Priority: 13.08.2021 US 202163232999 P
(71) Applicant: Welch Allyn, Inc., Skaneateles Falls, NY 13153-0220 (US)
(72) Inventor: KELLNER, David L, Skaneateles Falls, 13153-0220 (US); HUNTER, Vivian Loomis, Skaneateles Falls, 13153-0220 (US)
(74) Representative: Findlay, Alice Rosemary

(57) **Abstract**

A vision screening device for administering vision screening tests, and in particular a color vision screening test, to a patient is described herein. The vision screening device may include associated methods and systems configured to perform the operations of the vision screening tests. The device may include a first radiation source configured to generate color stimuli, a second radiation source separate from the first radiation source configured to emit near-infrared radiation, and a sensor configured to capture the near-infrared radiation emitted by the second radiation source, and reflected by an eye of a patient. The device may also be configured to cause color stimulus to be displayed to the patient, and determine measurement(s) of the eye of the patient in response to the color stimulus. The device may be further configured to analyze the measurements to generate a recommendation and/or diagnosis associated with the vision of the patient. The device may also be configured to display the recommendation and/or the measurements, along with additional screening data, to an operator conducting the vision test.

## Description

This application is directed to medical equipment. In particular, this application is directed to a vision screening device, and associated systems and methods, for assessment of color vision and detection of color vision disorders.

Visual screening in children and adults typically includes one or more tests to determine various deficiencies associated with the patient's eyes. Such vision tests may include, for example, refractive error tests, accommodation tests, visual acuity tests, color vision screening and the like. Conventional color vision screening tests present printed pseudoisochromatic plates (e.g., Ishihara or modified Ishihara plates) that embed numerals, characters of the alphabet, or shapes to the patient. The patient is expected to provide feedback on the visibility of the embedded content in the plates. The color vision status of a patient is determined based on the patient's feedback. The color vision status may include normal color vision, or the presence of a color vision deficiency. The type of color vision deficiency may also be determined from the test results.

Color vision may also be screened using a specialized device called anomaloscope. This device is also based on a patient's ability to provide feedback on whether two colors match. This method is typically used for further clinical evaluation of patients who have been identified as having a color vision deficiency based on the pseudoisochromatic plates-based tests.

The conventional color vision screening tests rely upon a patient's feedback upon viewing the color stimuli being presented to them. As a result, these tests are unsuitable for subjects who are unable to communicate or cooperate due to young age or a disability, as well as other uncooperative patients. Some studies have shown that approximately 8% of males and 0.5% of females exhibit some form of color vision deficiency. Though most types of color vision deficiency cannot be corrected, color vision screening is commonly administered so that the patient is made aware of any color vision deficiency, and can adjust for the impact of the deficiency on learning and performance of certain tasks.

Most vision tests are executed using a vision screening device, which may include ophthalmic testing devices such as a phoropter, autorefractor and photo-refractors. The use of printed or digitally displayed plates to screen for color vision requires an additional step in the vision screening process that adds to the time and complexity of the screening. It would be advantageous to be able to screen for most vision problems using a single integrated device.

The various examples of the present disclosure are directed toward overcoming one or more of the deficiencies noted above.

In an example of the present disclosure, a vision screening device includes a first radiation source configured to generate color stimuli, a second radiation source separate from the first radiation source, and a sensor configured to capture radiation emitted by the second radiation source, and reflected by an eye of a patient. The vision screening device also includes a processor operably connected to the first radiation source, the second radiation source, and the sensor, and a memory storing instructions executable by the processor. The instructions when executed, cause the processor to cause the first radiation source to present a first color stimulus, and a second color stimulus different from the first color stimulus, to the patient during a period of time, cause the second radiation source to emit near-infrared radiation during the period of time, and cause the sensor to capture, during the period of time, first near-infrared radiation reflected by the eye and responsive to the first color stimulus, and second near-infrared radiation reflected by the eye and responsive to the second color stimulus. The instructions, when executed, also cause the processor to determine a first measurement associated with the eye and based on the first near-infrared radiation, determine a second measurement associated with the eye and based on the second near-infrared radiation, determine a difference between the first measurement and the second measurement, determine that the difference is less than a threshold value; and generate, based at least in part on determining that the difference is less than the threshold value, a recommendation associated with the patient.

In another example of the present disclosure, a vision screening device includes a housing, a first display disposed on a first side of the housing and configured to generate color stimuli, a second display disposed on a second side of the housing opposite the first side, a radiation source configured to emit near-infrared (NIR) radiation, and a sensor configured to capture NIR radiation, emitted by the radiation source, and reflected by an eye of a patient. The vision screening device also includes a processor operably connected to the first display, the second display, the radiation source, and the sensor, and memory storing instructions that, when executed by the processor, cause the processor to cause the first display to present a first color stimulus, and a second color stimulus different from the first color stimulus, to the patient during a period of time, cause the radiation source to emit near IR during the period of time, and cause the sensor to capture, during the period of time, first near-infrared radiation reflected by the eye and responsive to the first color stimulus, and second near-infrared radiation reflected by the eye and responsive to the second color stimulus. The instructions, when executed, also cause the processor to determine a first refractive error based on the first near-infrared radiation, determine a second refractive error based on the second near-infrared radiation, determine a difference between the first refractive error and the second refractive error, determine that the difference is less than a threshold value, and generate, based at least in part on determining that the difference is less than the threshold value, a recommendation associated with the patient.

In still another example of the present disclosure, a method includes causing a first radiation source to present a first color stimulus, and a second color stimulus different from the first color stimulus, to a patient during a period of time, causing a second radiation source to emit near-infrared radiation during the period of time, and causing a sensor to capture, during the period of time, first data indicative of near-infrared radiation reflected by an eye of a patient and responsive to the first color stimulus, and second data indicative of near-infrared radiation reflected by the eye of the patient and responsive to the second color stimulus. The method also includes determining a first measurement associated with the eye and based on the first data, determining a second measurement associated with the eye and based on the second data, determining a difference between the first measurement and the second measurement, determining that the difference is less than a threshold value; and generating, based at least in part on determining that the difference is less than the threshold value, a recommendation associated with the patient.

The invention will now be further described by way of example with reference to the accompanying drawings, in which:.
FIG. 1 illustrates an example system of the present disclosure. In some implementations, components of the example system shown in FIG. 1 may be used to perform one or more tests associated with vision screening and/or detection of color vision deficiency.
FIG. 2 provides a schematic illustration of an example vision screening system of the present disclosure.
FIG. 3A illustrates an isometric view of an example vision screening device of the present disclosure.
FIG. 3B illustrates a cross-sectional view of the example vision screening device of the present disclosure.
FIG. 4 provides a schematic illustration of components of the example vision screening device of the present disclosure.
FIGS. 5A, 5B, and 5C illustrate additional examples of the vision screening device of the present disclosure.
FIGS. 6A, 6B, and 6C illustrate further examples of the vision screening device of the present disclosure.
FIG. 7 provides a first flow diagram illustrating an example method of the present disclosure.
FIG. 8 provides a second flow diagram illustrating an example method of the present disclosure.

In the figures, the left-most digit(s) of a reference number identifies the figure in which the reference number first appears. The use of the same reference numbers in different figures indicates similar or identical items or features. The drawings are not to scale.

The present disclosure is directed to, in part, a vision screening device, and corresponding methods. Such an example vision screening device may be configured to perform one or more vision screening tests on a patient and to output the results of the vision screening test(s) to an operator of the device, such as a physician or a physician's assistant. Specifically, the present disclosure is directed to devices and methods for a color vision screening test. For example, the vision screening device may generate one or more color stimuli, such as a series of color dots, color images, figures with color fringing, or other items useful for testing color vision capability of the patient. While the patient is viewing the color stimuli, the device may determine one or more measurements, such as a refractive error, pupil position, pupil size, and/or gaze angle, associated with one or more eyes of the patient.

Based at least in part on the measurements determined while the patient is viewing the color stimuli, the device may generate an output including at least one of a recommendation or a diagnosis associated with the patient. Such an output (e.g., the recommendation and/or the diagnosis) may be indicative of the color vision capability of the patient, e.g., whether the patient has normal color vision, requires additional screening, the type of color vision deficiency exhibited by the patient, and the like. For example, the device may compare the measurement(s) determined during the color vision screening test to standard testing data corresponding to normal color vision to provide the recommendation and/or diagnosis. In particular, the standard testing data may provide one or more thresholds or a range of values, and the output generated by the device may be based on the measurement(s) being less than the threshold(s) or being within the range of values. The output generated by the device may be displayed to the operator of the vision screening device. As such, the methods described herein may provide an automated diagnosis to assist the physician or other user of the vision screening device. The methods described herein may also provide an automated recommendation based on and/or indicative of such a diagnosis.

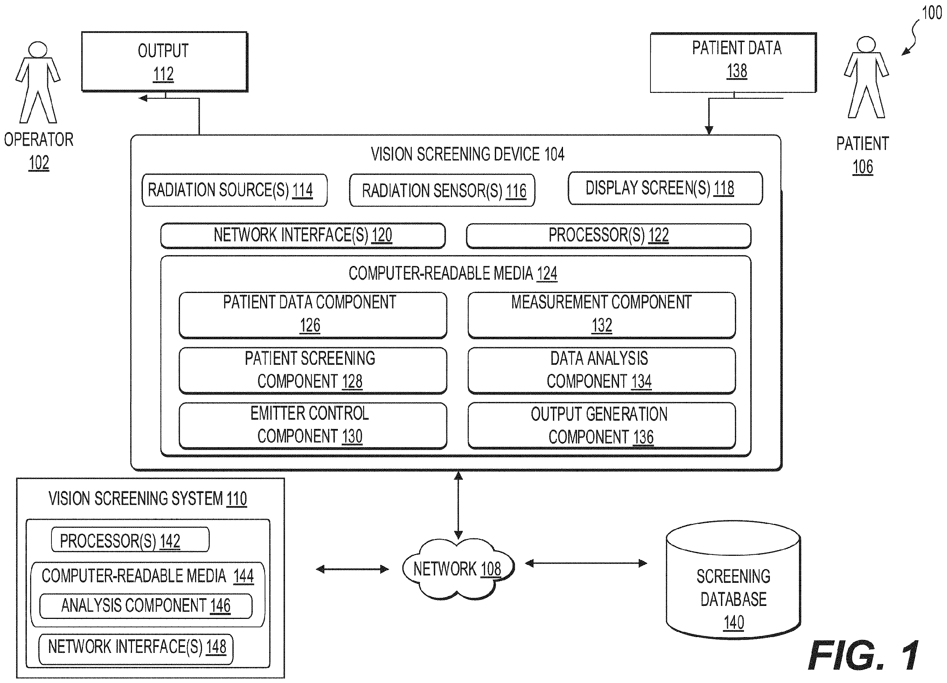

As will be described with respect to at least FIG. 1, an example vision screening device associated with screening for color vision deficiency may include components for generating a plurality of color stimuli that, when viewed in sequence, are expected to induce a change in one or more measurement(s) of the eye(s) of a patient with normal color vision, in response to some or all of the changes in color stimuli. The device may further include components for determining the measurement(s) in the eye(s) of the patient in response to viewing the color stimuli, and components for determining and reporting of output(s) of the screening test(s).

Additional details pertaining to the above-mentioned devices and techniques are described below with reference to FIGS. 1-8. It is to be appreciated that while these figures describe devices and systems that may utilize the claimed methods, the methods, processes, functions, operations, and/or techniques described herein may apply equally to other devices, systems, and the like.

FIG. 1 illustrates an example environment 100 for administering vision screening tests, and in particular, a color vision screening test, according to some implementations. As illustrated in FIG. 1, the environment 100 includes an operator 102 administering the vision screening tests, via a vision screening device 104, on a patient 106 to determine vision health of the patient 106. As described herein, the vision screening device 104 may perform one or more vision screening tests, including the color vision screening test. For example, in addition to the color vision screening test, the vision screening device 104 may also be configured to perform a visual acuity test, a refractive error test, an accommodation test, dynamic eye tracking tests, and/or any other vision screening tests, configured to evaluate and/or diagnose the vision health of the patient 106. In examples, the vision screening device 104 may comprise a portable device configured to perform the one or more vision screening tests. Due to its portable nature, the vision screening device 104 may perform the vision screening tests at any location, from conventional screening environments, such as schools and medical clinics, to physician's offices, hospitals, eye care facilities, and/or other remote and/or mobile locations.

As described herein, the vision screening device 104 may be configured to perform the color vision screening test on the patient 106. In examples, the color vision screening test may include displaying, in sequential order, a plurality of color stimuli, such as colored light patterns or images, configured to elicit an ocular response that causes a change in one or more measurements associated with eye(s) of the patient 106. In examples, the measurement(s) may comprise a measurement of refractive error of the eye(s) of the patient 106. For example, U.S. Patent No. 9,237,846, the entire disclosure of which is incorporated herein by reference, describes systems and methods for determining refractive error based on photorefraction using pupil images captured under different illumination patterns generated by near-infrared (NIR) radiation sources. During the administration of the color vision screening test, the vision screening device 104 may detect pupils, retinas, and/or lenses of the eye(s) of the patient 106, acquire data comprising images and/or video data of the pupils/retinas/lenses, and the like. This data may also be used to determine a measurement of gaze angle or gaze direction of the eye(s) of the patient, which may be tracked over time to determine a pattern of gaze angles or gaze directions during the color vision screening test. The vision screening device 104 may transmit the data, via a network 108, to a vision screening system 110 for analysis to determine an output 112 associated with the patient 106. Alternatively, or in addition, the vision screening device 104 may perform some or all of the analysis locally to determine the output 112. Indeed, in any of the examples described herein, some or all of the disclosed methods may be performed in whole or in part by the vision screening device 104, or by the vision screening system 110 separate from the vision screening device 104. For instance, in such examples, the vision screening device 104 may be configured to perform any of the vision screening tests, color vision tests, and/or other methods described herein without being connected to, or otherwise in communication with, the vision screening system 110 via the network 108.

As shown schematically in FIG. 1, the vision screening device 104 may include one or more radiation sources 114 configured to perform functions associated with administering the color vision screening test. The radiation source(s) 114 may comprise individual radiation emitters, such as light-emitting diodes (LEDs), which may be arranged in a pattern to form an LED array. In examples, the radiation source(s) 114 may include near-infrared (NIR) radiation emitters, such as NIR LEDs, for measuring the refractive error of the eye(s) of the patient 106 using photorefraction methods. The NIR radiation emitters of the radiation source(s) 114 may also be used for measuring the gaze angle or gaze direction of the eye(s) of the patient 106. In addition, the radiation source(s) 114 may include color LEDs for generating the plurality of color stimuli for display to the patient 106 during the color vision screening test. The radiation source(s) 114 may also include other emitters responsible for generating visible and/or infrared radiation for performing one or more of the other vision screening tests, in addition to the color vision screening test.

The vision screening device 104 may also include one or more radiation sensor(s) 116, such as cameras, configured to capture reflected radiation from the eye(s) of the patient during the vision screening test(s), including the color vision screening test. For example, the vision screening device 104 may emit, via the radiation source(s) 114, one or more beams of radiation, and may be configured to direct such beams at the eye(s) of the patient 106. The vision screening device 104 may then capture, via the radiation sensor(s) 116, corresponding radiation that is reflected back e.g., from pupils of the eye(s). Data corresponding to the reflected radiation captured by the radiation sensor(s) 116 may be used for determining the refractive error(s) and/or gaze angle(s) associated with the eye(s) of the patient 106. In examples, the radiation sensor(s) 116 may comprise NIR radiation sensor(s) to capture reflected NIR radiation while the eye(s) of the patient 106 are illuminated by the NIR radiation source(s) 114. The data captured by the NIR radiation sensor(s) 116 may be used in the measurement of the refractive error and/or gaze angle(s) of the eye(s) of the patient 106. The data may include images and/or video of the pupils, retinas, and/or lenses of the eyes of the patient 106. The data may be captured intermittently, during specific periods of the color vision or other vision screening test, or during the entire duration of the test(s). Additionally, the vision screening device 104 may process the image(s) and/or video data to determine change(s) in the refractive error and/or gaze angle(s) of the eye(s) of the patient 106. In examples, the reflected radiation from the patient's pupils may pass through a view window facing the patient 106, to reach the radiation sensor(s) 116.

The vision screening device 104 may also include one or more display screen(s) 118, such as a color LCD (liquid crystal display), or an OLED screen. The display screen(s) 118 may include an operator display screen facing a direction towards the operator 102, configured to provide information related to the vision screening tests to the operator 102. In any of the examples described herein, the operator display screen 118 facing the operator 102 may be configured to display and/or otherwise provide the output 112 generated by the vision screening device 104 and/or generated by the vision screening system 110. The output 112 may include testing parameters, current status of the test, measurements(s) determined during the test, a diagnosis determined based on one or more tests, and/or a recommendation associated with the diagnosis. The display screen 118 facing the operator 102 may also display information related to or unique to the patient, and the patient's medical history.

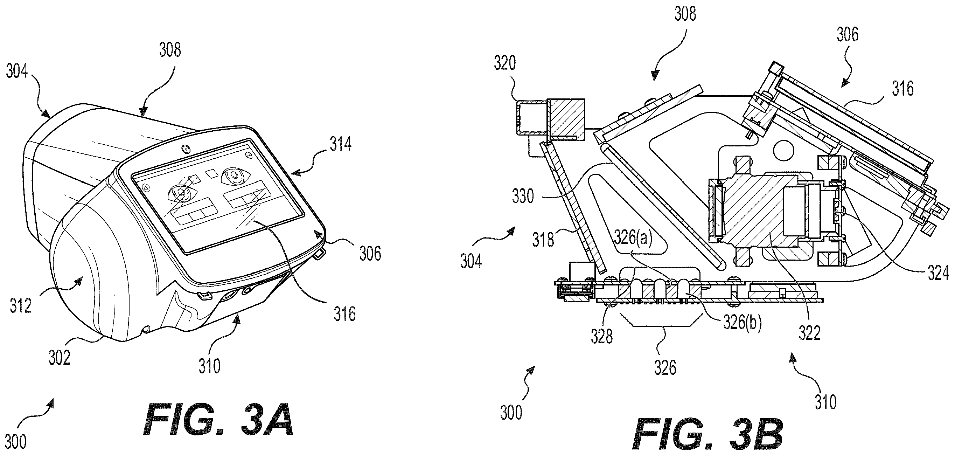

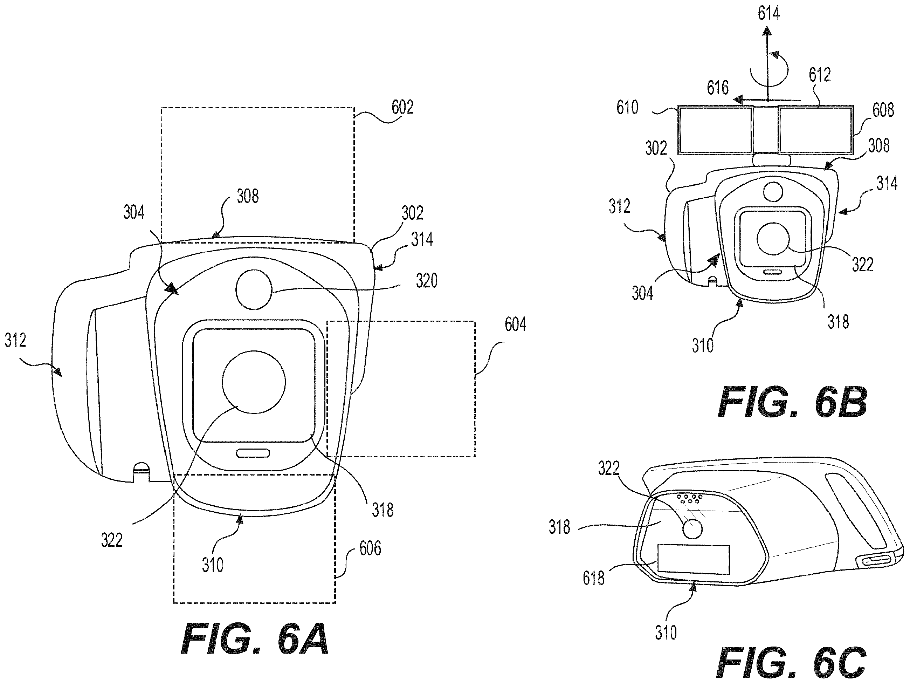

In some examples, the display screen(s) 118 may also include one or more display screens facing in a direction towards the patient 106, and configured to display the plurality of color stimuli to the patient 106. The color stimuli may be provided to the patient by images displayed on the display screen(s) 118 facing the patient. The display screen(s) 118 for providing the color stimuli may be integrated with the vision screening device 104, or may be external to the device, and under computer program control of the device. Additional details of the display screen(s) 118 associated with the vision screening device 104, will be discussed with reference to at least FIGS. 3A, 3B, and 6A-6C.

The vision screening device 104 may transmit the data captured by the radiation sensor(s) 116, via the network 108, using network interface(s) 120 of the vision screening device 104. In addition, the vision screening device 104 may also similarly transmit other testing data associated with the vision screening test(s) being administered, e.g., type of test, duration of test, patient identification and the like. The network interface(s) 120 of the vision screening device 104 may be operably connected to one or more processor(s) of the vision screening device 104, and may enable wired and/or wireless communications between the vision screening device 104 and one or more components of the vision screening system 110, as well as with one or more other remote systems and/or other networked devices. For instance, the network interface(s) 120 may include a personal area network component to enable communications over one or more short-range wireless communication channels, and/or a wide area network component to enable communication over a wide area network. In any of the examples described herein, the network interface(s) 120 may enable communication between, for example, the processor(s) 122 of the vision screening device 104, and the vision screening system 110, via the network 108. The network 108 shown in FIG. 1 may be any type of wireless network or other communication network known in the art. Examples of network 108 include the Internet, an intranet, a wide area network (WAN), a local area network (LAN), and a virtual private network (VPN), cellular network connections and connections made using protocols such as 802.11a, b, g, n and/or ac.

The vision screening system 110 may be configured to receive data, from the vision screening device 104 and via the network 108, collected during the administration of the vision screening test(s), such as the color vision screening test. In some examples, based at least in part on processing the data, the vision screening system 110 may determine the output 112 associated with the patient 106. For example, the output 112 may include a recommendation and/or diagnosis associated with color vision capability of patient 106, based on an analysis of the data indicative of the refractive error(s) and/or gaze angle(s) associated with the eye(s) of the patient 106 in response to the plurality of color stimuli presented during the color vision test. The vision screening system 110 may communicate the output 112 to the one or more processor(s) 122 of the vision screening device 104 via the network 108. As noted above, in any of the examples described herein one or more such recommendations, diagnoses, or other outputs may be generated, alternatively or additionally, by the vision screening device 104.

As described herein, a processor, such as processor(s) 122, can be a single processing unit or a number of processing units, and can include single or multiple computing units or multiple processing cores. The processor(s) 122 can be implemented as one or more microprocessors, microcomputers, microcontrollers, digital signal processors, central processing units, state machines, logic circuitries, and/or any devices that manipulate signals based on operational instructions. For example, the processor(s) 122 can be one or more hardware processors and/or logic circuits of any suitable type specifically programmed or configured to execute the algorithms and processes described herein. As shown schematically in FIG. 1, the vision screening device 104 may also include computer-readable media 124 operably connected to the processor(s) 122. The processor(s) 122 can be configured to fetch and execute computer-readable instructions stored in the computer-readable media 124, which can program the processor(s) 122 to perform the functions described herein.

The computer-readable media 124 may can include volatile and nonvolatile memory and/or removable and non-removable media implemented in any type of technology for storage of information, such as computer-readable instructions, data structures, program modules, or other data. Such computer-readable media 124 can include, but is not limited to, RAM, ROM, EEPROM, flash memory or other memory technology, optical storage, solid state storage, magnetic tape, magnetic disk storage, RAID storage systems, storage arrays, network attached storage, storage area networks, cloud storage, or any other medium that can be used to store the desired information and that can be accessed by a computing device. The computer-readable media 124 can be a type of computer-readable storage media and/or can be a tangible non-transitory media to the extent that when mentioned, non-transitory computer-readable media exclude media such as energy, carrier signals, electromagnetic waves, and signals per se.

The computer-readable media 124 can be used to store any number of functional components that are executable by the processor(s) 122. In examples, these functional components comprise instructions or programs that are executable by the processor(s) 122 and that, when executed, specifically configure the one or more processor(s) 122 to perform actions associated with one or more of the vision screening tests, such as the color vision screening test. For example, the computer-readable media 124 may store one or more functional components for administering the color vision screening test, such as a patient data component 126, a patient screening component 128, an emitter control component 130, a measurement component 132, a data analysis component 134, and/or an output generation component 136, as illustrated in FIG. 1. At least some of the functional components of the vision screening system 110 will be described in detail below.

In examples, the patient data component 126 may be configured to store and/or access data associated with the patient 106. For example, the patient 106 may provide data, such as patient data 138, upon initiating a vision screening test. For instance, when the vision screening device 104 and/or vision screening system 110 initiates a vision screening test, the patient 106 may provide, or the operator 102 may request, the patient data 138 regarding the patient's demographic information, disability information, preferences, and the like. For example, the patient 106 may provide demographic information such as name, age, ethnicity, and the like. In such examples, the operator 102 may request the data while the screening is in progress, or before the screening has begun. In some examples, the operator 102 may be provided with predetermined categories associated with the patient 106, such as predetermined age ranges (e.g., six to twelve months, one to five years old, etc.), and may request the patient data 138 in order to select the appropriate category associated with the patient 106. In other examples, the operator 102 may provide a free form input associated with the patient data 138. In still further examples, an input element may be provided to the patient 106 directly.

Alternatively, or in addition, the vision screening device 104 and/or vision screening system 110 may determine and/or detect the patient data 138 during the vision screening test. For example, the vision screening device 104 may be configured to generate image and/or video data associated with the patient 106 at the onset of the vision screening test. For example, the vision screening device 104 may include one or more digital cameras, motion sensors, proximity sensors, or other image capture devices configured to collect images and/or video data of the patient 106, and one or more processors of the vision screening device 104 may analyze the data to determine the patient data 138, such as the distance of the patient 106 from the screening device. For example, the vision screening device 104 may be equipped with a range finder, such as an ultra-sonic range finder, an infrared range finder, and/or any other proximity sensor that may be able to determine the distance of the patient 106 from the screening device.

Alternatively, or in addition, the vision screening device 104 may be configured to transmit the images/video data to the vision screening system 110, via the network 108, for analysis to determine the patient data 138. For example, the vision screening device 104 may transmit the image/video data to the vision screening system 110 and the patient data component 126 may be configured to analyze the data to determine the patient data 138. Still further, the patient data component 126 may be configured to receive, access, and/or store the patient data 138 associated with the patient 106 and/or additional patients. For example, the patient data component 126 may store previous patient information associated with the patient 106 and/or other patients who have utilized the vision screening system 110. For instance, the patient data component 126 may store previous patient preferences, screening history, and the like. The patient data component 126 may receive the patient data 138 and/or may access such information via the network 108. For example, the patient data component 126 may access an external database, such as screening database 140, storing data associated with the patient 106 and/or other patients. The screening database 140 may be configured to store the patient data 138 stored in association with a patient ID. When the operator 102 and/or patient 106 enters the patient ID, the patient data component 126 may access or receive the patient data 138 stored in association with the patient ID and the patient 106.

In examples, the computer-readable media 124 may also store a patient screening component 128. The patient screening component 128 may be configured to determine the plurality of color stimuli for display, in a sequence, to the patient 106, via the vision screening device 104, during the color vision screening test. A library of color stimuli for use the color vision screening test may be stored in the screening database 140 or be available on the computer-readable media 124, 144. The color stimuli may be based on psychophysical characteristics of human color vision, and configured to elicit differences in ocular response between patients with normal color vision and patients exhibiting types of color vision deficiency. The library may include different types of color stimuli, such as black figures including a color fringe displayed on a white background, graphics of a first color on a background of a second color different from the first color, color dot patterns, time-varying color images and the like.

The patient screening component 128 may be configured to receive and/or access the patient data 138 from the patient data component 126 to determine the color stimuli to display to the patient 106. As an example, the patient screening component 128 may utilize the patient data 138 to determine a testing category that the patient 106 belongs to (e.g., a testing category based on age, disability, etc.). Based on the patient data 138, and/or the testing category, the patient screening component 128 may determine the plurality of color stimuli for display to the patient 106 from the library of color stimuli. For example, if the patient data 138 indicates that the patient is a toddler, the color stimuli may comprise color dot graphics, whereas if the patient data 138 indicates an older patient who is able to read, the color stimuli may include text on a white background exhibiting various color fringing or colored text on a different colored background.

In any of the examples described herein, the patient screening component 128 may determine a sequence of color stimuli that transition between colors that the patient may find hard to distinguish. The plurality of color stimuli may follow a sequence that checks for different types of color vision deficiency that may be exhibited by the patient. For example, there are two main types of red-green color blindness, Protan and Deutan. A patient with protanomaly, or Protan type color-blindness, may have difficulty perceiving differences between red and black. Such a patient may also fail to perceive the color purple and the color pink. For example, to detect protanomaly, the patient screening component 128 may include a color stimulus that presents red graphics against a black background. In another example, the patient screening component 128 may include a purple dot pattern, followed by a pink dot pattern color stimulus in the plurality of color stimuli. In a patient with protanomaly, there may be small or no measurable change in the ocular response as these colors are not easily distinguishable by the patient. In a patient with deuteranomaly, or Deutan type color-blindness, the colors green, yellow, orange, red and brown may appear similar, especially in low light. It can also be difficult to differentiate between blues and purples, or pinks and grays. Based on these known symptoms of deuteranomaly, the patient screening component 128 may determine a sequence of color stimuli that transition between colors that the patient may find hard to distinguish e.g., a blue color stimulus to a purple color stimulus, in order to detect the condition. Other conditions may be similarly screened for by including in the plurality of color stimuli, a color stimulus, or a sequence of color stimuli, that would cause a response that is different from normal color vision in a patient exhibiting the condition. For example, tritanomaly may be screened for by transitions from blue to green, and transitions from red to purple. The patient screening component 128 may include a color stimulus or a sequence of color stimuli for each type of color vision deficiency being screened for. The patient screening component 128 may determine the sequence, and a duration of display for each color stimulus of the plurality of color stimuli.

In some examples, the color vision capability of the patient may be determined based on changes in the refractive error of the patient's eye(s) in response to changes in color stimuli being presented to the patient. The normal human eye focuses light in the red, blue and green channels on different parts of the eye, using the differences in focus of the three color channels to determine if the eye is in focus, and adjusting the focusing mechanisms of the eye as needed. Therefore, in a patient of normal color vision, the refractive error measurement(s) of the eye may change when subjected to a change in color stimuli designed to elicit such a change in refractive error (e.g., the refractive error may change by 0.25 or 0.5 diopters). Alternatively, or in addition, the gaze angle of the eye(s) may be indicative of the color vision capability the patient. The gaze angle of the eye(s) can be tracked to evaluate if the patient can see a color stimulus being presented. For example, if the patient is able to perceive different features in the color stimuli being presented, they may fixate on the features following a pattern of gaze angles. However, if the patient has some form of color vision deficiency, their eyes may not be able to perceive differences between different color stimuli, and therefore, exhibit no change or limited change in the refractive error measurement(s), and may exhibit a random pattern of gaze angles or shorter gaze time. The changes in refractive error measurement(s) may be compared with threshold(s) and/or range of values from standard testing data, as discussed, to determine whether the changes indicate a normal color vision response. The type of color vision deficiency (e.g., Protan or Deutan red-green color blindness, Tritanomaly blue color blindness, etc.) may also be determined from the specific change(s) in color stimuli for which the change in refractive error measurement(s) in the patient's eye(s) are less than the threshold(s), or outside the range of values, for normal color vision. Similarly, the pattern of gaze angles exhibited by the patient may be compared with standard gaze angle patterns corresponding to a normal color vision response, to determine whether the pattern of gaze angles indicate a normal color vision response.

In some examples, the computer-readable media 124 may additionally store an emitter control component 130. The emitter control component 130 may be configured to operate the radiation source(s) 114 of the vision screening device 104. As discussed, the radiation source(s) 114 may include NIR LEDs for measuring the refractive error and/or gaze angle of the eye(s) of the patient 106, and/or color LEDs for generating the color stimuli for display to the patient 106. In examples, the emitter control component 130 may generate commands to operate and control the individual radiation sources, such as LEDs, of the color LEDs and the NIR LEDs. Control parameters of the LEDs may include intensity, duration, pattern and cycle time. For example, the commands may selectively activate and deactivate the individual color LEDs of the radiation sources 114 to produce the plurality of color stimuli indicated by the patient screening component 128. In alternative examples, where the plurality of color stimuli is provided by images displayed on display screen(s) 118 visible to the patient 106, the emitter control component 130 may control the intensity, duration, pattern and cycle time of the images displayed on the display screen. The emitter control component 130 may activate the NIR LEDs of the radiation source(s) 114 used for measuring the refractive error and/or gaze angle of the eye(s) of the patient 106 in synchronization with the presentation of the color stimuli during the performance of the color vision screening test.

The individual radiation sources, such as LEDs, of the radiation source(s) 114 may be controlled by the emitter control component 130 according to control parameters stored in the computer-readable media 124. For instance, control parameters may include intensity, duration, pattern, cycle time, and so forth, for the color LEDs and/or the NIR LEDs of the radiation source(s) 114. For example, with respect to intensity, the control parameters may direct the color LEDs to emit light that is bright enough to attract attention of the patient 106, while also limiting brightness to avoid pupil constriction. Further, the emitter control component 130 may use the control parameters to display color stimuli such as color dot patterns to the patient 106 using the color LEDs of the radiation source(s) 114. These patterns may include circular patterns, alternating light patterns, flashing patterns, patterns of shapes such as circles or rectangles, and the like. The emitter control component 130 may also use the control parameters to determine a duration that individual LEDs of the radiation source(s) 114 emit radiation (e.g., 50 milliseconds, 100 milliseconds, 200 milliseconds, etc.). Additionally, the emitter control component 130 may utilize the control parameters to alter an intensity and display pattern of NIR LEDs of the radiation source(s) 114 for the determination of refractive error of the eye(s) based on photorefraction and/or gaze angle of the eye(s).

In some examples, the computer-readable media 124 may additionally store a measurement component 132. In such examples, the measurement component 132 may be configured to activate the radiation sensor(s) 116 of the vision screening device 104 and thereby cause the radiation sensor(s) 116 to capture data. The measurement component 132 may also be configured to analyze the data collected, detected, and/or otherwise captured by components of the vision screening device 104 (e.g., by the radiation sensor(s) 116) during one or more vision screening tests. The measurement component 132 may analyze the data to determine one or more measurements associated with the patient 106, such as an accommodation of a lens of the eyes of the patient 106, motion information associated with the eyes of the patient 106, the refractive error of the eye(s) of the patient 106, gaze angle of the eye(s) of the patient 106, and the like. For example, U.S. Patent Application No. 16/522,028, filed on July 25, 2019, and incorporated herein in its entirety, describes systems and methods for evaluating vision of a patient using image/video data captured by a sensor of a vision screening device. Any of the methods described in U.S. Patent Application No. 16/522,028 may be performed by the measurement component 132 in examples of the present disclosure.

For example, the measurement component 132 may be configured to receive, from the vision screening device 104, data, such as image data and/or video data captured by the radiation sensor(s) 116 during the color vision screening test. The data may be acquired while the plurality of color stimuli is being presented to the patient 106 by the vision screening device 104. In some examples, the measurement component 132 may determine the refractive error of one or both eyes of the patient 106, and/or a change in the refractive error of the eye(s), while the patient 106 is viewing each of the plurality of color stimuli. For example, the plurality of color stimuli displayed during the color vision screening test may include a first color stimulus, followed in the sequence by a second color stimulus. The measurement component 132 may receive, as part of the data, a first data captured during the period of presentation of the first color stimulus, and a second data captured during the period of presentation of the second color stimulus. Additionally, the data may include a third data that is captured during the transition period between the presentation of the first color stimulus and the second color stimulus. The measurement component 132 may determine the refractive error of the eye(s) based on the first data, and the refractive error of the eye(s) based on the second data, and may also determine a change in the refractive error responsive to the change in color stimuli from the first stimulus to the second stimulus. Alternatively, or in addition, the third data captured during the transition from the first color stimulus to the second color stimulus may be used to determine a change in the refractive error due to the change in color stimuli.

In another example, the measurement component 132 may be configured to determine a gaze direction or gaze angle of the eye(s) of the patient 106 in response to viewing the plurality of color stimuli being presented to the patient 106. For example, the gaze angle of the patient 106 may be determined by activating the radiation source(s) 114, such as the NIR LEDs, and directing the radiation emissions in the direction of the patient's 106 eye(s). In response, the cornea of the patient's 106 eye(s) may reflect the radiation, and the reflected radiation may be captured by the radiation sensor(s) 116. The measurement component 132 may utilize the image data and/or video data captured by the radiation sensor(s) 116 to determine a glint, or straight-line measurement, from the source of the light to the center of the eye (e.g., the origin of the reflection). As such, the measurement component 132 may utilize this information to determine a position, location, and/or motion of the pupil at different points in time during the presentation of the plurality of color stimuli. In other examples, the measurement component 132 may utilize the image/video data to determine the position or location of the pupil relative to the outside edges of the eye (e.g., the outline of the eye). The measurement component 132 may utilize the position and motion of the pupils of the eye(s) to determine the gaze angle of the eye(s) of the patient 106 during the presentation of the plurality of color stimuli.

Further, the computer-readable media 124 may also store a data analysis component 134. The data analysis component 134 may be configured to receive, access, and/or analyze standard testing data associated with vision testing. For example, the data analysis component 134 may be configured to access or receive data from one or more additional databases (e.g., the screening database 140, a third-party database, etc.) storing testing data, measurements, and/or values indicating various thresholds or ranges within which testing values should lie. Such thresholds or ranges may be associated with patients having normal vision health with similar testing conditions, and may be learned or otherwise determined from standard testing. The data analysis component 134 may utilize the standard testing data for comparison against the measurement(s) generated by the measurement component 132 described above. For example, the standard testing data associated with the color vision screening test may indicate a threshold or a range, where the change in the refractive error in the patient's eye(s) responsive to a change in color stimuli presented to the patient is greater than the threshold, or is within the range, when normal color vision is exhibited. Alternatively or in addition, the standard testing data may also indicate a pattern of gaze angles expected in response to the change in color stimuli in a patient exhibiting normal color vision. The data analysis component 134 may compare the gaze angle measurement(s) generated by the measurement component 132 with the indicated standard pattern of gaze angles to determine whether normal color vision is being exhibited. The comparison may be based on variability of gaze angles, duration of fixation of gaze angles, location of fixation points, alignment of the gaze angle with the direction of the color stimulus, and the like. Separate threshold(s) and/or range(s) may be indicated for different types of measurement(s). In addition, the threshold and/or range may be the same for each transition from a color stimulus to a subsequent color stimulus of the plurality of color stimuli, or may be different for different transitions. For instance, the transition from a first color stimulus to a second color stimulus may be associated with a first threshold and/or range, whereas the transition from the first color stimulus to a third color stimulus may be associated with a second threshold and/or range, different from the first threshold and/or range. The threshold(s) and/or range(s) associated with the color vision screening test may also be based on the testing category of the patient 106 e.g., the age group or disability status of the patient 106, where the threshold(s) and/or range(s) may be different for different testing categories. The data analysis component 134 may store patient data, measurements associated with vision screening tests, test results, and other data in a database(e.g., in the screening database 140) for comparison of data over time to monitor vision health status and changes in vision health.

Based on the comparison with a threshold and/or range described above, the data analysis component 134 may generate a pass/fail determination for each transition in the plurality of color stimuli displayed in a sequence to the patient 106. For example, if the change in refractive error determined by the measurement component 132 in response to a change in color stimuli exceeds a threshold value or falls within a range of the standard testing data, a pass determination may be made by the data analysis component 134, and a fail determination made otherwise. Alternatively, or in addition, the data analysis component 134 may generate a pass/fail determination for each color stimulus in the plurality of color stimuli displayed to the patient, based on the measured pattern of gaze angles matching the pattern of gaze angles indicating a normal color vision response to the color stimuli.

As discussed, a recommendation and/or a diagnosis associated with the color vision capability of the patient can be determined by comparing the measurements of refractive error of the patient's eyes when viewing different color stimuli. In examples, the system may compare the measurements with standard, or predetermined, measurements known to be associated with normal color vision capability. For example, criteria such as known thresholds and/or range of values of changes in the refractive error associated with normal color vision, may be compared with the measurements obtained during the color vision screening test, to determine whether the patient's eye(s) exhibit deficiencies related to color vision. In other examples, measurements of gaze angles obtained during the color vision screening test may be used to determine whether the patient exhibits a pattern of gaze angles corresponding to a normal color vision response. Depending on whether the measurements satisfy the criteria for normal color vision, the system may generate a diagnosis and/or recommendation associated with the patient. For example, if the measurements satisfy the criteria, the system may generate a recommendation indicating that the patient has passed the vision screening test. If the measurements do not satisfy the criteria, the system may generate a recommendation including an indication that the patient has failed the screening, an indication of a diagnosis of a type of color vision deficiency exhibited by the patient, or a recommendation for additional screening. In examples, the system may also utilize one or more machine learning techniques to generate the diagnosis recommendation. The recommendation, diagnosis and/or the measurements, may be presented to the operator of the device via an interface of the device. For example, the recommendation may be displayed to the operator on a display screen 118 of the vision screening device 104. In examples, the operator display screen may not be visible to the patient, e.g., the operator display screen may be facing in a direction opposite the patient. The operator display screen may also display information related to the vision screening tests, patient data, progress of the screening, and/or measurements obtained during the screening to the operator.

The computer-readable media 124 may additionally store an output generation component 136. The output generation component 136 may be configured to receive, access, and/or analyze data from the data analysis component 134. For example, the output generation component 136 may utilize the pass/fail determinations of the data analysis component 134 to determine if the patient 106 is exhibiting "normal" color vision. For example, a patient with normal color vision, also known as a "trichromat" vision uses three types of light cones in the eye(s) and can typically perceive up to one million different shades of colors. The data analysis component may generate an output 112 that may include a diagnosis associated with the color vision capability of the patient and/or a recommendation for further action(s). For example, a fail determination for one or more of the transitions in color stimuli may indicate an abnormality in color vision. In case an abnormality is determined, the specific transitions between the color stimuli for which the patient's measurement data resulted in a fail determination, may be used to further determine a type of color vision deficiency that is being exhibited by the patient 106. If normal color vision is determined, the output generation component 136 may generate output 112 indicating that the patient 106 has passed the color vision screening test. Alternatively, if abnormality is determined, the output generation component 136 may generate output 112 indicating that the patient 106 has failed the color vision screening test and/or indicating that the patient 106 should receive additional screening. The type of color vision deficiency determined may also be indicated in the output 112. The output 112 may be displayed to the operator 102 on a display screen(s) 118 of the vision screening device 104, or a screen associated with the color vision screening test e.g., on a computer display screen.

Although FIG. 1 illustrates example processor(s) 122, computer-readable media 124, a patient data component 126, a patient screening component 128, an emitter control component 130, a measurement component 132, a data analysis component 134, an output generation component 136 and/or other components and/or other items as components of the vision screening device 104, in any of the examples described herein, the vision screening system 110 may include similar components and/or the same components. In such examples, the vision screening system 110 may include processor(s) 142 and computer-readable memory 144 that are configured to perform the functions of some or all of the components in the computer-readable memory 124 of the vision screening device 104. For example, one or more of the components of the computer-readable memory 124 may be included in analysis component 146 of computer-readable memory 144 and be executable by the processor(s) 142. In such examples, the vision screening system 110 may communicate with the vision screening device 104 using network interface(s) 148, and via the network 108, to receive data from the vision screening device 104 and send results, e.g., output 112, back to the vision screening device 104. The vision screening system 110 may be implemented on a computer proximate the vision screening device 104, or may be at a remote location. For example, the vision screening system 110 may be implemented as a cloud service on a remote cloud server.

The network interface(s) 148 may enable wired and/or wireless communications between the components and/or devices shown in system 100 and/or with one or more other remote systems, as well as other networked devices. For instance, at least some of the network interface(s) 148 may include a personal area network component to enable communications over one or more short-range wireless communication channels. Furthermore, at least some of the network interface(s) 148 may include a wide area network component to enable communication over a wide area network. Such network interface(s) 148 may enable, for example, communication between the vision screening system 110 and the vision screening device 104 and/or other components of the system 100, via the network 108.

It should be understood that, while FIG. 1 depicts the system 100 as including a single vision screening system 110, in additional examples, the system 100 may include any number of local or remote vision screening systems substantially similar to the vision screening system 110, and configured to operate independently and/or in combination, and configured to communicate via the network 108.

As discussed herein, FIG. 1 depicts an exemplary vision screening device 104 that includes components for administering a color vision screening test to a patient. In some examples, one or more components may be implemented on a remote vision screening system 110 communicating with the vision screening device 104 over a network 108. The vision screening device 104 and its components are described in detail with reference to the remaining figures.

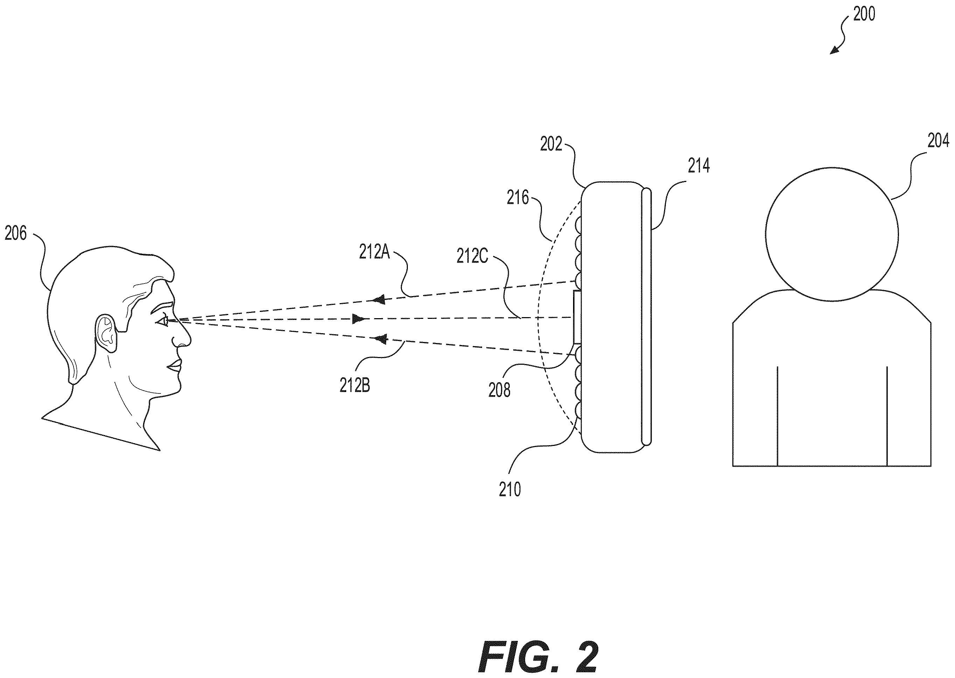

FIG. 2 illustrates an example system 200 including a schematic illustration of components of a vision screening device 202, which may be substantially similar to or the same as the vision screening device 104 of FIG. 1, according to examples of the present disclosure. In examples, an operator 204 may utilize the vision screening device 202 and/or other components of the system 200 to administer a color vision test to a patient 206. As shown in FIG. 2, the vision screening device 202 may include radiation sources 210, which may be substantially similar to or the same as radiation source(s) 114, and sensor(s) 208, which may be substantially similar to or the same as the radiation sensor(s) 116. The radiation source(s) 210 may be configured to emit radiation in the visible band and/or the near-infrared (NIR) band. For instance, the radiation sources 210 may comprise an arrangement of differently-colored LEDs that emit visible radiation. The color LEDs may be configured to generate the plurality of color stimuli used in the color vision screening test. The radiation sources 210 may also comprise NIR LEDs configured to determine the refractive error and/or the gaze angle associated with one or more eyes of the patient 206 while the patient 206 views the plurality of color stimuli.

The radiation sources 210 may emit a plurality of radiation beams, including radiation beam 212A and 212B, configured to illuminate the eye(s) of the patient 206. The reflected visible and/or NIR radiation 212C from the eye(s) of the patient 206 may be captured via the sensor(s) 208. The vision screening device 202 may detect the pupils and/or lenses of the eyes of the patient 206 and/or acquire images and/or video data of the pupils/lenses via the sensor(s) 208. In examples, the NIR radiation 212C may be used to capture, via the sensor(s) 208, pupil images to determine the refractive error and/or the gaze angle of the eye(s) of the patient 206. Examples of capturing pupil images and determining corresponding refractive errors associated with the eyes of the patient are described in the disclosure of, for example, U.S. Patent No. 9,237,846, referred to above and incorporated herein by reference.

The sensor(s) 208 may include optical components, such as one or more lenses, windows, prisms, filters, mirrors, and/or any other devices configured to collect and direct the radiation beams generated by the radiation sources 210, including radiation beam 212A and 212B, to the eye(s) of the patient 206. In some further examples, the optical components may also comprise a collimating lens, a convergent, lens, a divergent lens, and/or any other substantially transparent lens or series of lenses configured to assist in directing the reflected beam 212C from the eye(s) of the patient 206 to impinge the sensor(s) 208. In examples, the radiation beams, such as beams 212A, 212B, and the reflected beam 212C from the patient's pupils may pass through a transparent view window 216 facing the patient. Non-optical components of the vision screening device 202 may include, for example, an operator display screen 214. It is noted that the vision screening device 202 is not limited to the components listed here, and may incorporate additional components for furthering vision screening techniques.

As described herein, FIG. 2 illustrates components of the vision screening device 202, including optical components involved in the generation and targeting of radiation from radiation source(s) to the eye(s) of the patient, as well as the targeting of reflected radiation from the eye(s) of the patient to sensor(s) configured to capture the reflected radiation.

FIG. 3A illustrates an embodiment of a vision screening device 300 according to some implementations, and FIG. 3B illustrates a cross-sectional view of the vision screening device 300 illustrated in FIG. 3A. The example vision screening device 300 includes some or all of the components described above with reference to the vision screening device 104 of FIG. 1 and/or the vision screening device 202 of FIG. 2. The vision screening device 300 may include a housing 302, with a first end 304 and a second end 306 opposite the first end 304. The housing 302 may also have a top surface 308, a bottom surface 310 opposite the top surface 308, a first side 312 (e.g., a left side), and a second side 314 (e.g., a right side) opposite the first side 312, as shown. The first end 304 may be configured to face a patient e.g., patient 106 or 206, and the second end 306 may be configured to face an operator of the vision screening device 300 e.g., operator 102, 204. For example, the second end 306 may include a display screen 316 configured to provide information and/or other output 112 to the operator. The display screen 316 may comprise, for example, a liquid crystal display (LCD) or active matrix organic light emitting display (AMOLED). The display screen 304 may also be touch-sensitive to receive input from the operator of the vision screening device 300.

As shown in FIG. 3B, the vision screening device 300 may also include a view window 318, which may be substantially similar to the view window 216 of the vision screening device 202. The plurality of radiation beams 212A, 212B as well as the reflected radiation beam 212C may pass through the view window 318, which may be transparent. Other optical components of the vision screening device 300 may include, for example, a range finder 320, and a lens component 322 coupled to a sensor 324. The vision screening device 300 may also include a light-emitting diode (LED) array 326 with individual LEDs 326(a) and 326(b) which may emit radiation at different wavelengths, a diffuser 328, and a beam splitter 330. In examples, LEDs 326(a) may be color LEDs, while LEDs 326(b) may be near-infrared (NIR) LEDs. The diffuser 328 may cause the radiation emitted by the LEDs 326(a), 326(b) to be output to the view window 318 at a desired combined intensity, configured to reduce or inhibit accommodation of the eye(s) of the patient. The radiation may be reflected by the eyes(s) of the patient, and return through the view window 318, be transmitted through the lens component 322 to be received by sensor 324 of the vision screening device 300. Raw sensor data from sensor 324 may be processed e.g., to enhance contrast or transform to a display format, for presentation and feedback to an operator of the device via the display screen 316. Additional information may also be displayed on the display screen 316, such as a distance of the patient from the vision screening device 300 which may be obtained by the range finder 320, quality of focus, progress of the examination, and/or other information related to the vision screening examination process.

In some examples, the view window 318 may also function as a display screen facing towards the patient, which may be transparent to allow radiation to pass through without change. Alternatively, or in addition, only a portion of the view window 318 may be transparent to allow radiation to pass through to reach the sensor 324, while the remaining portion of the view window 318 may comprise a non-transparent display screen. The display screen may be used to present visual stimuli, such as the plurality of color stimuli used during the color vision screening test, to the patient. It is noted that the vision screening device 300 is not limited to the components listed here, and may incorporate more or fewer components for furthering vision screening techniques.

In some examples, the sensor 324 includes, for example, a complementary metal-oxide semiconductor (CMOS) sensor array, also known as an active pixel sensor (APS), or a charge connected device (CCD) sensor. In some examples, the lens component 322 is supported by the vision screening device 300 and positioned in front of the sensor 324. In still further examples, the sensor 324 has a plurality of rows of pixels and a plurality of columns of pixels. For example, the sensor 324 may include approximately 1280 by 1024 pixels, approximately 640 by 480 pixels, approximately 1500 by 1152 pixels, approximately 2048 by 1536 pixels, and/or approximately 2560 by 1920 pixels. The sensor 324 may be capable of capturing approximately 25 frames per second (fps), approximately 30 fps, approximately 35 fps, approximately 40 fps, approximately 50 fps, approximately 75 fps, approximately 100 fps, approximately 150 fps, approximately 200 fps, approximately 225 fps, and/or approximately 250 fps. Note that the above pixel values and frames per second are exemplary, and other values may be greater or less than the examples described herein.

In examples, the sensor 324 may include photodiodes having a light-receiving surface and have substantially uniform length and width. During exposure, the photodiodes convert the incident radiation to a charge. The sensor 324 may be operated as a global shutter. For example, substantially all of the photodiodes may be exposed simultaneously and for substantially identical lengths of time. Alternatively, the sensor 324 may be used with a rolling shutter mechanism, in which exposures move as a wave from one side of an image to the other. Other mechanisms are possible to operate the sensor 324 in yet other examples. The sensor 324 may also be configured to capture digital image data. The digital image data can be captured in various formats, such as JPEG, BITMAP, TIFF, etc.

As discussed herein, FIG. 3 illustrates an exemplary vision screening device 300, including components of the device configured to generate color and near-infrared radiation directed to the eye(s) of the patient, capture reflected radiation from the eye(s) of the patient, and display output information to an operator of the device 300. The vision screening device 300, as shown, is a portable device thereby allowing for usage at different types of locations such as schools, physician's offices, hospitals and/or other remote and/or mobile locations.

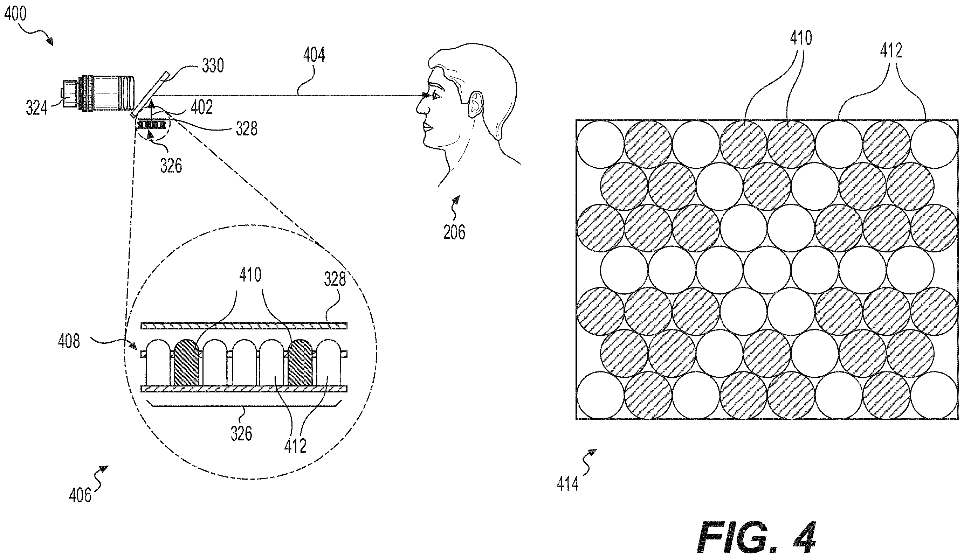

FIG. 4 illustrates an example system 400 including components of the vision screening device 300 according to examples of the present disclosure. The example system 400 illustrates the sensor 324, the LED array 326, the diffuser 316, and the beam splitter 318 of FIG. 3B. Other components of the vision screening device 300 have been omitted in the example system 400 for clarity.

In examples, the radiation 402 emitted by one or more LEDs in the LED array 326 passes through the diffuser 316 and strikes the beam splitter 318. The diffuser 316 may act as a blur smoothing filter for light emitted by the LEDs in the LED array 326. At least a portion 404 of the radiation 402 reflects off of the beam splitter 318 and is directed to one or more eyes of the patient 206. While the portion 404 of the radiation 402 is directed at the eye(s) of the patient 206, the sensor 324 captures one or more images and/or video of the eye(s) of the patient 206. In examples, the image(s) and/or video may depict radiation that is reflected by the pupil(s) of the eye(s) of the patient 206 e.g., for use in the determination of the refractive error and/or the gaze angle of the eye(s).

An expanded view 406 of FIG. 4 illustrates the LED array 326 and the diffuser 316 of the vision screening device 300, depicting a row 408 of the LED array 326. The LED array 326 may be arranged with color LEDs 410, which may correspond to LEDs 314(a), positioned between and coplanar with NIR LEDs 412, which may correspond to LEDs 314(b), as shown in the expanded view 406. The row 408 includes color LEDs 410 and NIR LEDs 412 in an example arrangement. A plan view 414 illustrates additional details of an example arrangement of color LEDs 410 and NIR LEDs 412 in the LED array 326 from an orientation above the LED array 326. In some cases, the LED array 326 may include more or fewer individual LEDs than those shown in the example orientation. The configuration of the LED array 326 with color LEDs 410 and NIR LEDs 412 allows the color stimuli for the color vision screening test to be presented independently of NIR LED radiation used in determining refractive error and/or the gaze angle, while using the same optical components, such as the diffuser 328, the beam splitter 330, and the lens component 322 associated with the sensor 324.

As discussed with reference to FIG. 4, color LEDs 410 used for generating the plurality of color stimuli may be embedded in the LED array 326 along with the NIR LEDs 412. The LED array 326 may be disposed within a housing 302 of the vision screening device, visible to the patient through the view window 318.

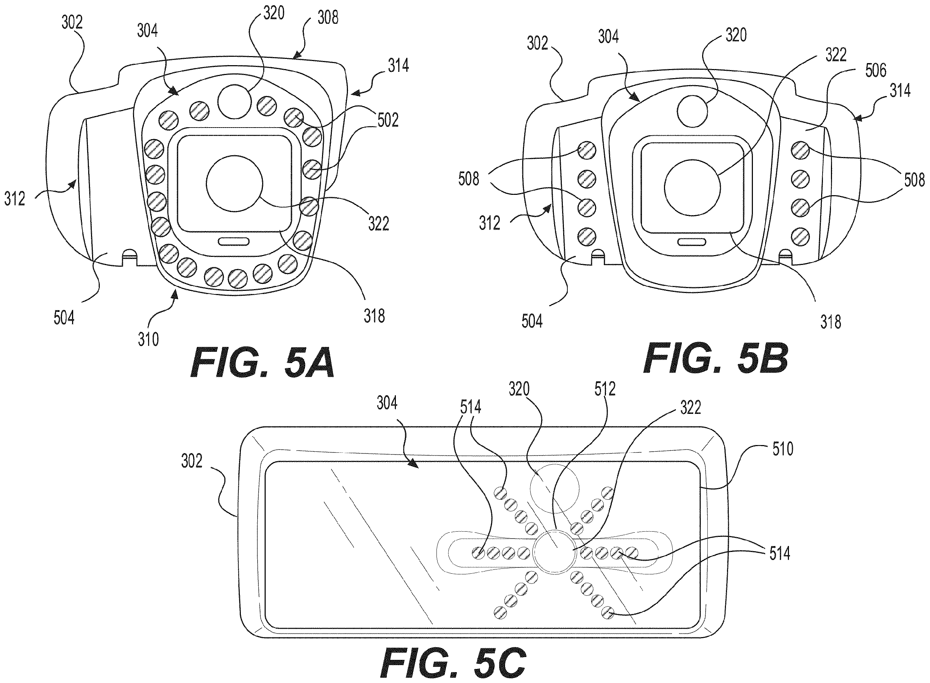

In alternative embodiments, the color LEDs may not be included in the LED array 326, and instead, be disposed on external surfaces of the housing 302 of the vision screening device 300. FIGS. 5A-C illustrates alternative arrangements of color LEDs for generating the plurality of color stimuli on the housing 302 of the vision screening device 300, showing the vision screening device 300 as viewed from the first end 304.

FIG. 5A illustrates an example arrangement of the color LEDs 502 surrounding the view window 318. As discussed with reference to FIG. 3B, the view window 318 is disposed at the first end 304 of the vision screening device 300 that faces the patient. The color LEDs 502 may be disposed on an area of the housing 302 between the view window 318 and the top surface 308, between the view window 318 and the bottom surface 310, between the view window 318 and the first side 312, and/or between the view window 318 and the second side 314, as shown in FIG. 5A. In this example, the LED array 326 may comprise only the NIR LEDs, or may include one or more color LEDs. The color LEDs 502 are disposed at the first end 304 facing towards the patient, so that the color stimuli presented using the color LEDs is visible to the patient. The NIR radiation reflected from the patient's eyes(s) during the color vision screening test passes through the view window 318 and into the lens component 322 that directs the reflected radiation to the sensor 324 (not shown) of the vision screening device 300, as described with reference to FIG. 3B.

In an alternative arrangement, the color LEDs 508 may be disposed on a side panel 504 on the first side 312 of the vision screening device 300, as shown in FIG. 5B. As also shown in FIG. 5B, the vision screening device 300 may also include an additional side panel 506 on the second side 31 of the device 300, and the color LEDs 508 may be disposed on the side panel 50. The color LEDs 508 are disposed on the first end 304 of the vision screening device 300 proximate the view window 318, which faces the patient. This arrangement of color LEDs 508 is thus visible to the patient. As discussed with reference to FIG. 5A, the LED array 326 (not shown) of the vision screening device 300 may include only NIRLEDs in this example arrangement.

FIG. 5C illustrates an alternative embodiment of the vision screening device 300. In the example shown, the view window 318 is replaced by a transparent display screen 510, such as a transparent organic light emitting display (OLED). The transparent display screen 510 faces the first end 304 of the vision screening device 300, facing the patient. The display screen 510 may cover an opening 512 in the housing 302 of the device 300 leading to the lens component 322. Since the display screen 510 is transparent, radiation reflected from the patient's eye(s) may travel to the lens component 322 through the opening 512, and through the display screen 510, without change. The color LEDs 514 may be distributed in a pattern around the opening 512 and disposed on the surface of the housing 302 facing the first end 304. Though a pattern radiating outwards from the opening 512 is shown, other patterns of placement of the color LEDs are also envisioned.

As discussed with reference to FIG. 5A, the LED array 326 (not shown) of the vision screening device 300 may not include color LEDs in the example device shown in FIG. 5C. In alternative examples, the LED array 326 may include both color LEDs and NIR LEDs as shown in FIG. 4, and the transparent display screen 510 may be a head-up display, reflecting radiation from color LEDs 410 in the LED array 326 directed towards the opening 512 by the beam splitter 330 as shown in FIG. 4. The reflected radiation from the color LEDs may be visible to the patient on the substantially vertical surface of the display screen 510. In such an alternative example, there may be no color LEDs 514 disposed on the housing 302 of the vision screening device 300.

As discussed, the plurality of color stimuli may be generated by color radiation sources, such as the color LEDs 514 or the color LEDs 410 of the LED array 326 as described above, or may be presented to the patient as color images displayed on a display screen, such as the display screen 510. In yet another alternative example, the vision screening device 300 shown in FIG. 5C may not have color LEDs 514 disposed on the housing 302 of the device 300, and instead, the plurality of color stimuli may be displayed to the patient on the transparent display screen 510.

In any of the examples described herein (e.g., with reference to any of the example display screen configurations described herein), the plurality of color stimuli displayed during the color vision screening test may include a first color stimulus that is a standard colored image without any color shift. Such a standard image should elicit a baseline response from a viewer, and the image may comprise a digital image illustrating one or more objects, in color. In such examples, the plurality of color stimuli may also include a second color stimulus that is color shifted (e.g., that has been modified to accent, highlight, or otherwise emphasize) toward a first color (e.g., red). For instance, the second color stimulus may comprise the same image illustrated in the first color stimulus, but the pixels, LEDs, or other components of the display screen used to illustrate the one or more objects in the image may be controlled by the processor(s) 122 and/or the display screen to accentuate existing red colors present in the image. Additionally or alternatively, the processor(s) 122 and/or the display screen may control such components to shift, transition, change, and/or otherwise modify colors characterized by wavelengths that are close to red wavelengths in the visible spectrum (e.g., orange colors, yellow colors, etc.) such that they appear red or at least partly red in the second color stimulus. In such examples, the degree to which such colors are modified may be directly related to the difference between the wavelength of the respective color and the wavelength of red light. It is understood that the wavelength of red light is between approximately 620 nm and approximately 750 nm. For instance, objects with colors having a wavelength closer to red wavelengths (e.g. orange light, having a wavelength between approximately 620 nm and approximately 590 nm) may be modified to appear more red in the second color stimulus relative to objects with other colors (e.g., yellow light, having a wavelength between approximately 590 nm and approximately 560 nm) having a wavelength further from the red wavelengths. Alternatively, in some embodiments objects with colors beyond a desired color threshold may be shifted or otherwise modified to have a different color. For example, objects with colors having a wavelength above approximately 590 nm (e.g., orange) may be modified, in whole or in part, to have a wavelength of approximately 700 nm (e.g., red).

In any of the examples described herein, the plurality of color stimuli may further include a third color stimulus that is color shifted toward a second color different from the first color (e.g., green). For instance, the third color stimulus may comprise the same image illustrated in the first color stimulus, but the pixels, LEDs, or other components of the display screen used to illustrate the one or more objects in the image may be controlled by the processor(s) 122 and/or the display screen to accentuate existing green colors present in the image. Additionally or alternatively, the processor(s) 122 and/or the display screen may control such components to shift, transition, change, and/or otherwise modify colors characterized by wavelengths that are close to green wavelengths in the visible spectrum (e.g., yellow colors, cyan colors, etc.) such that they appear green or at least partly green in the third color stimulus. Further, although described above as comprising the same image, it is understood that in further examples, the first color stimulus may comprise a digital image illustrating one or more objects, in color, and at least one of the second color stimulus or the third color stimulus may comprise a different digital image illustrating one or more different obj ects.

In any of the examples described herein, the measurement component 132 and/or the processor(s) 122 may receive first data captured by the one or more sensors 324 of the vision screening device during the period of presentation of the first color stimulus, second data captured during the period of presentation of the second color stimulus, and third data captured during the period of presentation of the third color stimulus. Additionally, the measurement component 132 and/or the processor(s) 122 may receive data captured by the one or more sensors 324 during the transition periods between the presentation of the first color stimulus, the second color stimulus, and/or the third color stimulus. The measurement component 132 and/or the processor(s) 122 may determine a first refractive error of the eye(s) based on the first data, a refractive error of the eye(s) based on the second data, and a third refractive error of the eye(s) based on the third data. The measurement component 132 and/or the processor(s) 122 may determine such first, second, and third refractive errors based on any of the processes described herein. The measurement component 132 and/or the processor(s) 122 may also determine a change in the refractive error responsive to the change in color stimuli. For instance, the measurement component 132 and/or the processor(s) 122 may determine a first difference between the first refractive error and the second refractive error. The measurement component 132 and/or the processor(s) 122 may also determine a second difference between the first refractive error and the third refractive error. The measurement component 132 and/or the processor(s) 122 of the vision screening device may also compare one or more such differences to corresponding difference thresholds indicative of normal color vision. For instance, when viewing the color stimuli described above, a patient with normal color vision may exhibit between at least an approximately ¼ and ½ diopter shift as between the first color stimulus and the second color stimulus. A patient with normal color vision may also exhibit between an approximately ¼ and ½ diopter shift as between the first color stimulus and the third color stimulus. It is understood that in some examples, other thresholds may be used. If the measurement component 132 and/or the processor(s) 122 determines that the first difference and the second difference are greater than or equal to the corresponding difference thresholds, the measurement component 132 and/or the processor(s) 122 may, based on such a determination, indicate or otherwise determine that the patient has normal color vision. On the other hand, if the measurement component 132 and/or the processor(s) 122 determines that the first difference and/or the second difference is less than the corresponding difference thresholds, the measurement component 132 and/or the processor(s) 122 may, based on such a determination, indicate or otherwise determine that the patient suffers from color blindness.

In some examples, the thresholds noted above may be selected based on the age, gender, ethnicity and/or other characteristics of the patient. Additionally, different thresholds may be selected based on the type of color stimulus presented to the patient. For example, a first threshold or set of thresholds may be employed when presenting red shifted image(s) as the second color stimulus. In such an example, a second threshold or set of thresholds may be employed when presenting green shifted image(s) as the third color stimulus. In this way, refractive error differences may be compared to corresponding red/green difference thresholds to determine whether the patient suffers from red/green color blindness. On the other hand, a different set of thresholds may be employed when presenting red/blue shifted images as the second and third color stimuli, respectively. Thus, the above process may be utilized to not only determine the presence of protanomaly, deuteranomaly, and/or tritanomaly, but depending on the thresholds used and the color shifting of the stimuli presented, the measurement component 132 and/or other computation components of the vision screening device may also be configured to determine the type of color deficiency detected.

FIG. 6A-C illustrate other example display screen configurations associated with the vision screening device 300. FIG. 6A indicates alternative positions of a display screen relative to the vision screening device 300 when viewed from the first end 304. For example, the vision screening device 300 may include a display screen 602 pivotably and/or otherwise connected to the top surface 308 of the device 300, above the view window 318 and facing the first end 304 towards the patient. The vision screening device 300 may also include, alternatively or in addition, a display screen 604 pivotably or otherwise connected to the second side 314 of the housing 302 of the device 300. In yet another example, the vision screening device 300 may include, alternatively or in addition, a display screen 606 pivotably or otherwise connected to the bottom surface 310 of the device 300, below the view window 318, and facing the first end 304 towards the patient. One or more of the display screens 602, 604, 606 may be present. The attachment of the display screen 602, 604, 606 to the housing 302 of the vision screening device 300 may be hinged and/or otherwise pivotable, so that the display screen 602, 604, 606 may be rotated with respect to the housing 302 of the device 300. For example, rotation of the display screen 602, 604, 606 in a first direction away from the housing 302 may allow the screen to be parallel to a plane of the view window 318, whereas rotation of the display screen 602, 604, 606 in a second direction toward from the housing 302 may bring the display screen 602, 604, 606 flush against and/or substantially parallel to the top surface 308, the second side 314, or the bottom surface 310 respectively of the housing 302. The hinged attachment described above allows the display screens 602, 604, 606 to be stowed flush against the housing 302 of the vision screening device 300 when not in use. The display screens 602, 604, 606 may also be retractable, so that they may retract into the housing 302 of the vision screening device 300 when not in use. In other examples, the display screens 602, 604, 606 may not be permanently attached to the vision screening device 300, and instead, may be removably attached to the housing 302 and/or may be communicatively coupled to the vision screening device 300. In any such examples, the example display screens 602, 604, 606 described herein may enable the display of the plurality of color stimuli to be controlled by components of the vision screening device 300.

Fig. 6B illustrates a display screen 608 that may be attached to the top surface 308 of the housing 302 of the vision screening device 300 as shown. The display screen 608 may include two separate component screens e.g., LCD screens, 610 and 612. The component screen 610 may be disposed towards the first side 312 of the vision screening device 300, and the component screen 612 may be disposed facing the second side 314 of the vision screening device 300. The display screen 608 may be pivotably connected to the top surface 308 of the vision screening device 300. The display screen 608 may be rotatable about the vertical axis 614 as shown. This arrangement may allow the display screen 608 to be rotated from facing the first end 304 (e.g., facing the patient), to facing the second end 306 (e.g., facing the operator) of the vision screening device 300. The component screens 610, 612 of the display screen 608 may be slidably connected to the housing 302, being configured to slide along a horizontal axis 616. For example, the component screen 612 may slide towards the first side 312, overlapping over the component screen 610 of the display screen 608. Alternatively, the component screen 610 may slide towards the second side 314 overlapping over the component screen 612. In yet another example, the component screen 610 may slide towards the second side 314, and the component screen 612 may slide towards the first side 312, where the component screens 610 and 612 overlap when viewed from the first end 304.

In some examples, the component screens 610 and 612 of the display screen 608 may display different content. For example, the component screen 610 may display the plurality of color stimuli, whereas the component screen 612 of the screen may display instructions or indicate a progress status of the color vision screening test. The component screens 610 and 612 of the display screen 608 may also be used to provide visual stimuli to the left and right eye of the patient respectively e.g., the component screen 610 may display visual stimulus directed to the left eye, whereas the component screen 612 may display visual stimulus directed to the right eye of the patient.

FIG. 6C illustrates another example of the vision screening device 300. As shown, a display screen 618 as shown is integrated with the view window 318. The display screen 618 may be used to display the plurality of color stimuli to the patient during the administration of the color vision screening test. The view window 318 is transparent, allowing radiation to pass through to the lens component 322 without change. In some examples, the display screen 618 may also be transparent. As discussed with reference to FIG. 5C, the transparent display screen 618 may be a configured as a head-up display, reflecting radiation from color LEDs or a separate display screen located inside the housing 302, the radiation from the color LEDs or the separate display screen being directed towards the display screen 618 by optical components such as mirrors and beam splitters of the vision screening device 300.

The display screens 602, 604, 606, 608, 618 shown in FIGS. 6A-6C may comprise, for example, a liquid crystal display (LCD), active matrix organic light emitting display (AMOLED), transparent OLED, and/or a flexible display such as flexible OLCD or flexible OLED. In all examples shown in FIG. 6A-C, the display screens 602, 604, 606, 608, 618 may also be used to display other content associated with vision screening tests other than the color vision screening test. For example, the display screens 602, 604, 606, 608, 618 may display content related to a visual acuity test e.g., numbers, letters or shapes at different sizes.

In various examples, as described herein with reference to FIGS. 5 and 6, the vision screening device 300 may include color LEDs and/or display screen(s) configured to display the plurality of color stimuli to a patient during the color vision screening test. As discussed, the color LEDs and/or display screen(s) may be arranged in various ways and disposed at the first end of the device facing the patient, so that the color stimuli are visible to the patient.

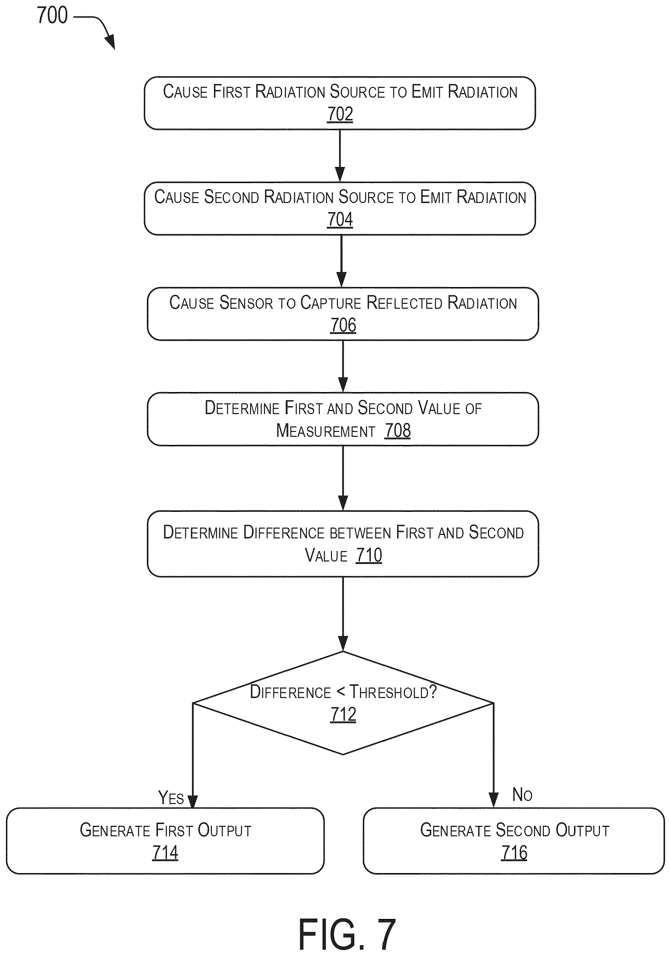

FIGS. 7-8 provide flow diagrams illustrating example methods for vision screening, as described herein. The methods in FIGS. 7-8 are illustrated as collections of blocks in a logical flow graph, which represents sequences of operations that can be implemented in hardware, software, or a combination thereof. In the context of software, the blocks represent computer-executable instructions stored on one or more computer-readable storage media that, when executed by processor(s), perform the recited operations. Generally, computer-executable instructions include routines, programs, objects, components, data structures, and the like that perform particular functions or implement particular abstract data types. The order in which the operations are described is not intended to be construed as a limitation, and any number of the described blocks can be combined in any order and/or in parallel to implement the methods illustrated in FIGS. 7-8. In some embodiments, one or more blocks of the methods illustrated in FIGS. 7-8 can be omitted entirely.

The operations described below with respect to the methods illustrated in FIGS. 7-8 can be performed by any of the devices or systems 100, 200, and 300 described herein, and/or by various components thereof. Unless otherwise specified, and for ease of description, the methods illustrated in FIGS. 7-8 will be described below with reference to the system 100 shown in FIG. 1, and the vision screening device 104, 202, 300, 400 of FIGS. 1-4. In particular, any of the operations described with respect to the methods illustrated in FIGS. 7-8 may be performed by the emitter control component 130, the measurement component 132, the data analysis component 134, and/or the output generation component 136 executed by the processor(s) 122 of the vision screening device 104, 202, 300, or 400, and/or by the analysis component 146 executed by the processor(s) 142 of the vision screening system 110, either alone or in combination.

FIG. 7 provides a flow diagram illustrating an example method 700 of the present disclosure. At operation 702, the emitter control component 130 and/or one or more processors associated therewith causes a first radiation source 114 to emit radiation. For example, the first radiation source 114 may comprise color LEDs of the radiation source(s) 114 of the vision screening device 104, configured to generate the plurality of color stimuli indicated by the patient screening component 128. The emitter control component 130 may select and activate the color LEDs of the radiation source(s) 114 to display a first color stimulus of the plurality of color stimuli, followed in sequence by a second color stimulus of the plurality of color stimuli that is different from the first color stimulus during a period of time corresponding at least in part to the administration of the color vision screening test.

At operation 704, the emitter control component 130 and/or one or more processors associated therewith causes a second radiation source, separate from the first radiation source, to emit radiation e.g., near-infrared (NIR) radiation. For example, the second radiation source may comprise NIR LEDs of the radiation source(s) 114 of the vision screening device 104, NIR LEDs 326(b) of FIG. 3, or NIR LEDs 412 of FIG. 4, configured to direct NIR radiation to the eye(s) of the patient for the measurement of refractive error(s) and/or the gaze angle(s) of the eye(s). The emitter control component 130 may select and set a pattern of NIR LEDs to activate for the measurement of refractive error(s) and/or the gaze angle(s). The activation of the NIR LEDs at operation 704 may occur while the patient views the first color stimulus and while the patient views the second color stimulus during the period of time corresponding at least in part to the administration of the color vision screening test. In some examples, the activation of the NIR LEDs at operation 704 may alternate with the activation of the color LEDs at operation 702. For example, the first radiation source may be activated for a first duration, the second radiation source may be activated for a second duration, alternately during the period of time.

The emitter control component 130 may also select and set an LED pattern of the LED array 326 for activation. In some examples, the arrangement of the NIR LEDs 326(b) in the LED array 326 allows for different illumination patterns to be presented to the eye(s) of the patient 106. The measurement accuracy of the refractive error of the eye(s) may depend upon the illumination pattern selected. Additional details regarding illumination patterns used in examination protocols for determining refractive error can be found in U.S. Patent Application No. 9,237,846, referred to above and incorporated herein by reference.

At operation 706, the measurement component 132 may activate radiation sensor(s) 116 of the vision screening device 104 to capture first NIR radiation reflected by the eye(s) of the patient and responsive to the first color stimulus, and second NIR radiation reflected by the eye(s) of the patient and responsive to the second color stimulus. For example, the first NIR radiation may be captured during the presentation of the first color stimulus, and the second NIR radiation may be captured during the presentation of the second color stimulus. The measurement component 132 may receive first data indicative of the first NIR radiation captured, and second data indicative of the second NIR radiation captured by the sensor e.g., radiation sensor(s) 116. The first data and the second data may include image(s) and or video of the eye(s) illuminated by NIR radiation. In some examples, the measurement component 132 may also determine a first pattern of gaze angles based on the first data, and a second pattern of gaze angles based on the second data.

At operation 708, the measurement component 132 may determine a first value of a measurement and a second value of a measurement associated with the eye(s) of the patient. In examples, as discussed, the measurement may be the refractive error of the eye(s). The measurement component 132 may determine a first value of the refractive error of the eye(s) from the first data indicative of the first NIR radiation, and a second value of the refractive error of the eye(s) from the second data indicative of the second NIR radiation, using the techniques described in U.S. Patent No. 9,237,846, referred to above and incorporated herein by reference, for determining refractive error from pupil images captured under NIR radiation illumination.

At operation 710, the measurement component 132 may determine a difference between the first value of the refractive error responsive to the first color stimulus, and the second value of the refractive error responsive to the second color stimulus. As discussed, in a patient of normal color vision, the refractive error measurement of the eye may change when subjected to a change in color stimuli e.g., by presenting the first color stimulus, followed by the second color stimulus different from the first color stimulus at operation 702. For example, the difference between the first value and the second value may be in the range of 0.25 to 0.5 diopters in some examples of a patient with normal color vision. The difference may be greater or less than this range based on individual differences and color vision capability of the patient.

At operation 712, the data analysis component 134 may compare the difference between the first value and the second value obtained at operation 710 with a threshold value to determine that the difference is less than the threshold value. For example, the threshold value may be predetermined and available as a part of standard testing data, which may be stored in the screening database 140 or the computer-readable media 124, 144. The threshold may correspond to a lower threshold of difference between values of the refractive error in the eye in response to a change in color stimulus from the first color stimulus to the second color stimulus. If the difference determined at operation 710 is less than the threshold value, the output generation component 136 may generate a first output associated with the patient at operation 714, and if the difference is equal to or higher than the threshold value, the output generation component 136 may generate a second output at operation 716. The first output at operation 714 may correspond to an indication that the patient has failed the color vision screening test, a recommendation of additional screening, and/or a diagnosis of a type of color vision deficiency determined by the first color stimulus and the second color stimulus. Whereas the second output at operation 716 may correspond to an indication of that the patient has passed the color vision screening, or that the patient exhibits normal color vision.

In an alternative example, where the pattern of gaze angles is measured to evaluate color vision capability as discussed, one or more operations of process 700 may be omitted or modified. For example, at operation 708, the first value of measurement may correspond to a pattern of gaze angles determined in response to a color stimulus generated by the first radiation source 114 at operation 702. In such examples, the pattern of gaze angles may be determined based on the reflected radiation captured by radiation sensor(s) 116 at operation 706. At operation 710, a difference may be determined (e.g., by the data analysis component 134) between the pattern of gaze angles and a standard pattern of gaze angles associated with normal color vision. Instead of comparing the difference to a threshold as indicated at operation 712, differences in variability of gaze angles, duration and location of fixation of gaze angles, alignment with the direction of the color stimulus, and the like may be analyzed by the data analysis component 134 to determine if a first output corresponding to an abnormality is generated at operation 714, or a second output corresponding to normal color vision response is generated at operation 716.

As discussed, the example process 700 may be performed by the components of the vision screening device 104 executed by the processor(s) 122 of the device 104. The example process 700 illustrates operations performed during at least a part of a color vision screening test administered to a patient. In alternative examples, some or all of the operations of process 700 may be executed by processor(s) 142 of a vision screening system 110 that is connected to the vision screening device 104 via network 108.

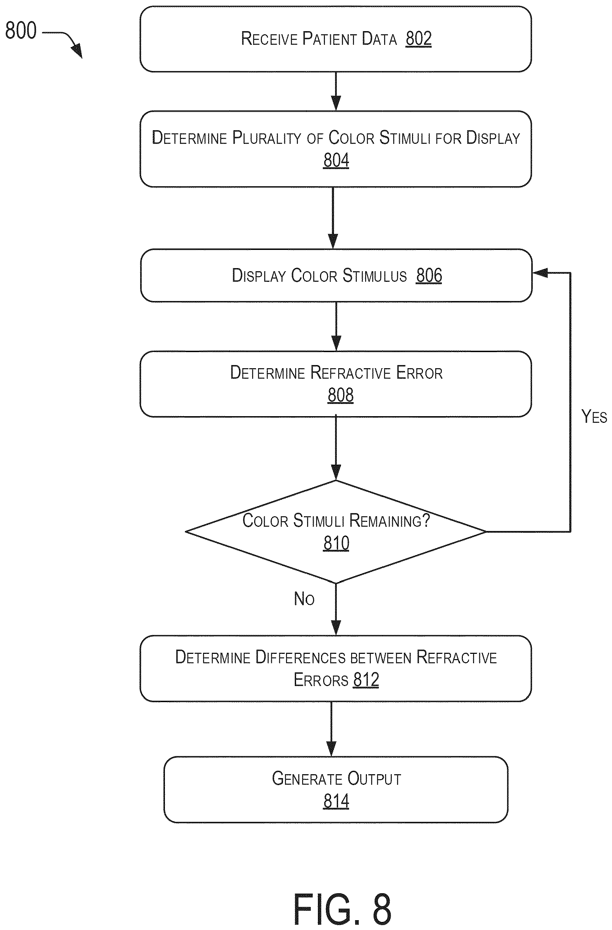

FIG. 8 illustrates an example process 800 for vision screening according to some implementations of the present disclosure. As discussed, the operations of the process 800 will be described as being performed by the processor(s) 122 the vision screening device 104, even though the operations may also be alternatively or additionally performed by the processor(s) 142 of the remote vision screening system 110.

At operation 802, the patient data component 126 may receive patient data 138 associated with a patient participating in a vision screening test, such as the color vision screening test. As described herein, a patient being evaluated by the vision screening system may provide information to the system. In some examples, the patient may manually enter such information via one or more touchscreens or other input devices of the vision screening device. In additional examples, a physician or other operator of the device may manually enter such information via such input devices. Additionally, or alternatively, the system may receive and/or access data associated with the patient from a database of the system storing information associated with patients, such as the screening database 140. The received and/or accessed data may be analyzed by the system to determine one or more characteristics associated with the patient, such as demographic information, physical characteristics of the patient, etc. Still further, in some examples, the system may generate image or video data associated with the patient and may analyze the image/video data to determine the characteristics associated with the patient.

At operation 804, the patient screening component 128 may determine a plurality of color stimuli for display. The plurality of color stimuli may be displayed, one at a time and in sequence, to a patient participating in a color vision screening test. As described herein, the patient data received at operation 802 may be utilized to determine a testing category associated with the patient e.g., a testing category based on age, gender, disability, and the like. The plurality of color stimuli for display to the patient may be selected from an existing library of color stimuli stored in a database of the system, such as the screening database 140, based on the patient data and/or the testing category associated with the patient. For example, the color stimuli may include color dot patterns, time varying color patterns, black figures including a color fringe on a white background, graphics of a first color on a background of a second color different from the first color, and the like. For example, color stimuli comprising color dot patterns may be determined if the testing category indicates a toddler patient, or time-varying color patterns may be selected for a patient with a disability involving short attention spans. For an adult patient who is able to read, the color stimuli selected for display may be text or other graphics with color fringing on a white background or colored text or other figures on a different colored background. In addition to the color stimuli, the database may also store a duration of display for each stimulus. The color stimuli and duration of display may be based on psychophysical characteristics of human color vision, and configured to elicit a difference in values of a measurement associated with the eye, such as a refractive error and/or a gaze angle, responsive to the change in color stimuli. In any of the examples described herein, and as described above with reference to any of the example display screen configurations described herein, at operation 804, the plurality of stimuli determined by the patient screening component 128 may include a first (e.g., baseline) color stimulus comprising an image illustrating an object, a second color stimulus modified to accentuate first (e.g., red) colors present in the image, and/or a third color stimulus modified to accentuate second (e.g., green) colors present in the image different from the first colors.

At operation 806, the vision screening device 104 may display a color stimulus from the plurality of color stimuli determined at operation 804. The color stimulus displayed may be the next color stimulus in the sequence, starting with the first color stimulus at the start of the vision screening test, and the color stimulus may be displayed for a duration indicated for the color stimulus at operation 804. The display of the color stimuli may be controlled by the emitter control component 130, and additional parameters of the display may include intensity, pattern, cycle time, and so forth. The color stimulus may be displayed on a display screen 118, such as an LCD or OLED screen, associated with the vision screening device 104. The display screen may be facing a direction towards the patient so that the display is visible to the patient. In alternative examples, the display of color stimuli comprising color dot patterns, or time-varying color dots, may be generated by color LEDs of the radiation source(s) 114 associated with the vision screening device 104. As described herein, the color LEDs may be embedded in an LED array, or distributed on a housing of the vision screening device, facing in a direction towards the patient. In any of the examples of color stimuli described herein, the color stimulus may be also be fogged or defocused. A defocused stimulus may reduce the ability of the patient's eye(s) to accommodate while viewing and/or focusing on the stimulus, which may result in a more accurate determination of the refractive error of the eye.

At operation 808, the measurement component 132 may determine the refractive error of the eye(s) of the patient responsive to the color stimulus displayed at operation 806. The refractive error may be determined based on characteristics of NIR radiation reflected from the pupil of the eye(s) of the patient, for example. As described herein, the radiation source(s) 114 of the vision screening device 104 may include NIR LEDs configured to emit NIR radiation directed to the eye(s) of the patient. The reflected radiation may be captured by a sensor of the radiation sensor(s) 116 of the vision screening device 104, and used for the determination of refractive error as described in U.S. Patent No. 9,237,846, referred to above and incorporated herein by reference.

At operation 810, the vision screening device 104 may determine if there are color stimuli remaining to be displayed from the plurality of color stimuli determined at operation 804. As discussed, each color stimulus of the plurality of color stimuli is to be displayed one at a time, in a sequence. The emitter control component 130 may keep track of the color stimulus that was displayed at operation 806, and an index indicating its position in the plurality of color stimuli. If the index of the color stimulus at operation 806 is at a last position in the plurality of color stimuli, then there are no further color stimuli remaining, and the process 800 moves to operation 812 as shown. If the position of the color stimulus at operation 806 is not the last position, then there are additional color stimuli remaining to be displayed, and the process 800 reverts back to operation 806 to display the next color stimulus in the sequence from the plurality of color stimuli.

At operation 812, the data analysis component 134 may determine differences between refractive errors obtained at operation 808 in response to the display of each of color stimulus, at operation 806, of the plurality of color stimuli. As described herein, the plurality of color stimuli is displayed one at a time and in sequence at operation 806, and a refractive error is determined for each color stimulus at operation 808. For example, a first refractive error may be determined responsive to a first color stimulus, and a second refractive error may be determined based on a second color stimulus, and a difference between the first refractive error and the second refractive error determined at operation 812. A difference or change in a value of refractive error is determined for the display of each color stimulus and the display of the subsequent color stimulus, for each transition of color stimulus in the sequential display of the plurality of color stimuli. In some examples, at operation 812, the data analysis component 134 and/or the processor may determine the presence of color blindness based on the determined differences. Additionally or alternatively, depending on the thresholds used and the color shifting of the stimuli presented, the measurement component 132, the data analysis component 134, and/or other computation components of the vision screening device may also determine the type of color deficiency detected. For instance, as described herein, and in examples in which a first (e.g., a baseline) color stimulus and a second color stimulus are presented, a first threshold or set of thresholds may be employed when presenting red shifted image(s) as the second color stimulus. If a third color stimulus is presented to the patient in such an example, a second threshold or set of thresholds may be employed when presenting green shifted image(s) as the third color stimulus. In this way, refractive error differences may be compared to corresponding red/green difference thresholds to determine whether the patient suffers from red/green color blindness. A similar process may be used, at operation 812, when testing for other types of color blindness.

At operation 814, the output generation component 136 generates an output based on the differences determined at operation 812. The data analysis component 134 may access standard testing data, e.g., from the screening database 140, indicating a threshold and/or range of change in refractive error corresponding to each transition of color stimulus to the subsequent color stimulus in the sequence, corresponding to normal color vision. Based on a comparison with the threshold and/or range established in the standard testing data, the data analysis component 134 may determine whether the difference in refractive error, determined at operation 812 for each transition, exceeds the threshold, or is within the range, indicated for normal color vision. If the difference is less than the threshold, or outside the range, an abnormality in color vision is determined. Otherwise, a normal color vision response is determined. If every transition of color stimulus is determined to produce a normal color vision response, e.g., the difference in refractive error measurement exceeds the threshold, or falls within the range of normal color vision, the output generated at operation 814 may indicate that the patient exhibits normal color vision, or that the patient has passed the vision screening test. Instead, if one or more of the transitions are determined to show abnormality, the output may indicate that the patient has failed the vision screening test. In this instance, the output may also include a recommendation that the patient needs further screening, or may include a diagnosis of a type of color vision deficiency based on the abnormalities determined. The output may be displayed to an operator of the vision screening device 104 on the display screen(s) 118.

Based at least on the description herein, it is understood that the vision screening devices and associated systems and methods of the present disclosure may be used to assist in performing one or more vision screening tests, including a color vision screening test. The components of the vision screening device described herein may be configured to determine and present a plurality of color stimuli to present to a patient undergoing vision screening, determine a measurement associated with the eye of the patient responsive to the color stimuli, and determine an output indicating a diagnosis, recommendation or results of the screening test. An exemplary vision screening device may include radiation source(s) and/or display screen(s) for generating the plurality of color stimuli, radiation source(s) and sensor(s) for generating near-infrared radiation for determining a refractive error of the eye of the patient, and display screen(s) for displaying the output to an operator of the vision screening device. The device described herein may be used for screening a patient for color vision deficiency without requiring inputs or feedback from the patient, thereby allowing the device to be used for screening young or uncooperative patients.

Embodiments of the invention can be described with reference to the following numbered clauses, with preferred features laid out in the dependent clauses:
1. A vision screening device, comprising:
   a housing;
   a first display disposed at a first end of the housing and configured to generate color stimuli;
   a second display disposed at a second end of the housing opposite the first end;
   a sensor configured to capture radiation reflected by an eye of a patient;
   a processor operably connected to the first display, the second display, and the sensor; and
   memory storing instructions that, when executed by the processor, cause the processor to:
      cause the first display to present a first color stimulus, and a second color stimulus different from the first color stimulus, to the patient during a period of time;
      cause the sensor to capture:
         first radiation reflected by the eye and responsive to the first color stimulus, and
         second radiation reflected by the eye and responsive to the second color stimulus;
      determine, based on the first radiation, a first value of a measurement associated with the eye;
      determine, based on the second radiation, a second value of the measurement;
      generate, based at least in part on the first value and the second value, an output comprising at least one of a diagnosis or a recommendation associated with the patient; and
      present the output via the second display.
2. The vision screening device of clause 1, wherein the first display is disposed below, adjacent to, or above a view window of the vision screening device, the first and second radiation reflected by the eye passing to the sensor via the view window.
3. The vision screening device of either clause 1 or clause 2, wherein the first display is transparent, and the first and second radiation reflected by the eye pass to the sensor via the first display.
4. The vision screening device of clause 3, wherein the first display generates the color stimuli by reflecting radiation from an LED array or a third display.
5. The vision screening device of any one of clauses 1 to 4, wherein the first and second color stimuli comprise at least one of:
   black figures on a white background, the black figures including a color fringe;
   graphics of a first color on a background of a second color different from the first color;
   color dot patterns; or
   time-varying color images.
6. A system, comprising:
   memory;
   a processor; and
   computer-executable instructions stored in the memory and executable by the processor to perform operations comprising:
      receiving patient data unique to a patient participating in a color-vision screening test;
      determining, based at least in part on the patient data, a plurality of color stimuli for presentation to the patient, the color stimuli being configured to change a measurement associated with an eye of the patient when the color stimuli is observed by the patient;
      causing the plurality of color stimuli to be presented to the patient during the color-vision screening test;
      determining one or more values of the measurement associated with the eye responsive to the plurality of color stimuli;
      determining whether the one or more values satisfy one or more thresholds; and
      generating an output associated with the patient, the output indicating whether the one or more values satisfy the one or more thresholds.
7. The system of clause 6, wherein the measurement is indicative of a refractive error of the eye.
8. The system of either clause 6 or clause 7, wherein the plurality of color stimuli comprises one or more of:
   black graphics on a white background, the black graphics including a color fringe;
   graphics of a first color disposed on a background of a second color different from the first color;
   color dot patterns; or
   time-varying color images.
9. The system of any one of clauses 6 to 8, wherein the output is indicative of at least one of passed screening, additional screening required, or a type of color vision deficiency.
10. A method, comprising:
   causing a first radiation source to present a color stimulus to a patient during a period of time, wherein the color stimulus comprises a color pattern including a first color and a second color;
   causing a second radiation source to emit near-infrared radiation during the period of time;
   causing a sensor to capture, during the period of time, reflected near-infrared radiation from an eye of the patient and responsive to the color stimulus;
   determining, based on the reflected near-infrared radiation, gaze direction of the eye during the period of time;
   determining a difference between the gaze direction of the eye during the period of time and a pre-determined gaze direction; and
   generating, based at least in part on the difference, an output indicative of an ability of the patient to distinguish between the first color and the second color of the color stimulus.

## Claims

1. A vision screening device, comprising:
a radiation source configured to generate color stimuli;
a sensor configured to capture radiation reflected by an eye of a patient;
a processor operably connected to the radiation source and the sensor; and
memory storing instructions that, when executed by the processor, cause the processor to:
cause the radiation source to present a first color stimulus, and a second color stimulus different from the first color stimulus, to the patient during a period of time;
cause the sensor to capture:
first radiation reflected by the eye and responsive to the first color stimulus, and
second radiation reflected by the eye and responsive to the second color stimulus;
determine, based on the first radiation, a first value of a measurement associated with the eye;
determine, based on the second radiation, a second value of the measurement; and
generate, based at least in part on the first value and the second value, an output indicative of an ability of the patient to distinguish between the first color stimulus and the second color stimulus.

2. The vision screening device of claim 1, wherein the radiation source is a first radiation source, the vision screening device further comprising a second radiation source configured to emit near-infrared (NIR) radiation, wherein the instructions further cause the processor to:
cause the second radiation source to emit NIR radiation during the period of time.

3. The vision screening device of either claim 1 or claim 2, wherein the radiation source comprises at least one of:
color light emitting diodes (LEDs);
an organic light emitting display (OLED) screen; or
a liquid crystal display (LCD) screen.

4. The vision screening device of claim 3, wherein the radiation source comprises at least one of color LEDs disposed proximate a perimeter of a view window, and the view window is disposed at a first end of the vision screening device, the first and second radiation reflected by the eye passing to the sensor via the view window.

5. The vision screening device of claim 4, further comprising a display unit disposed at a second end of the vision screening device opposite the first end, the display unit being configured to display the output associated with the patient.

6. The vision screening device of claim 2, wherein the radiation source is a first radiation source comprising color light emitting diodes (LEDs), the second radiation source comprises near-infrared (NIR) LEDs, and the color LEDs and the NIR LEDs are interspersed in a pattern on an LED array of the vision screening device.

7. The vision screening device of any preceding claim, wherein the measurement comprises a refractive error of the eye or a gaze direction of the eye.

8. The vision screening device of any preceding claim, wherein the first color stimulus comprises a first color pattern, and the second color stimulus comprises a second color pattern, the first and second color patterns configured to cause a difference between the first value and the second value when observed by a patient exhibiting trichromat color vision.

9. The vision screening device of any preceding claim, wherein the output includes an indication of: passed screening, additional screening required, or a type of color vision deficiency.

10. A method, comprising:
causing a first radiation source to present a first color stimulus, and a second color stimulus different from the first color stimulus, to a patient during a period of time;
causing a second radiation source to emit near-infrared radiation during the period of time;
causing a sensor to capture, during the period of time,
first near-infrared radiation reflected by an eye of the patient and responsive to the first color stimulus, and
second near-infrared radiation reflected by the eye of the patient and responsive to the second color stimulus; and automatically:
determining, based on the first near-infrared radiation, a first value of a measurement associated with the eye;
determining, based on the second near-infrared radiation, a second value of the measurement;
determining a difference between the first value and the second value;
determining that the difference is less than a threshold value; and
generating, based at least in part on determining that the difference is less than the threshold value, an output associated with the patient, the output indicating an ability of the patient to distinguish between the first color stimulus and the second color stimulus.

11. The method of claim 10, further comprising:
receiving patient data associated with the patient; and
determining, based at least in part on the patient data, the first and second color stimuli.

12. The method of either claim 10 or claim 11, wherein the first radiation source comprises one of:
color light-emitting diodes (LEDs); or
color display screen.

13. The method of any one of claims 10 to 12, wherein the measurement is indicative of a refractive error of the eye of the patient.

14. The method of any one of claims 10 to 13, wherein the first and second color stimuli comprise at least one of:
black figures on a white background, the black figures including a color fringe;
graphics of a first color on a background of a second color different from the first color;
color dot patterns; or
time-varying color images.

15. The method of any one of claims 10 to 14, further comprising displaying the output on a display screen, wherein the output is indicative of at least one of: passed screening, additional screening required, or a type of color vision deficiency.
